# EUROPEAN PATENT APPLICATION

(11) **EP 3 861 857 A1**
(43) Date of publication of application: **11.08.2021**
(21) Application number: 20156074.5
(22) Date of filing: 07.02.2020
(51) Int. Cl.: A01N 29/02, A01N 37/10, A01N 37/22, A01N 53/00, A01N 25/22

(54) **ANTIPARASITIC COMPOSITIONS**

(71) Applicant: Novaliq GmbH, 69120 Heidelberg (DE)
(72) Inventor: The designation of the inventor has not yet been filed
(74) Representative: Leo, Chiara Silvana

(57) **Abstract**

The present invention relates to an antiparasitic composition comprising an antiparasitic agent and a semifluorinated alkane.

## Description

The present invention relates to compositions and methods that can be used in the treatment of parasitic infestations, in particular ectoparasitic infestations of the skin.

### Background of the invention

The term infestation refers to parasitic diseases caused by animals such as arthropods (i.e. mites, ticks, and lice) and worms. An ectoparasitic infestation is a parasitic disease caused by organisms that live primarily on the surface of the host. Examples of ectoparasitic infestations are scabies, body lice, head lice, ticks and fleas.

Mites are among the smallest arthropods. The majority of mite species are harmless to humans or domestic animals, but a few species can colonise mammals and cause allergenic diseases. Many types of itchy skin rashes are caused by mites. Among these, scabies is caused by the parasitic mite *Sarcoptes scabiei.* Scabies is a contagious infestation transmitted by skin to skin contact and sometimes by contact with contaminated material. Scabies is transmitted by the female scabies mite, which burrows into the top layer of the epidermis to lay eggs, before dying 30 to 60 days later. If infestation occurs, the first papules appear within 2 to 5 weeks. The main symptom of scabies is a severe itch, which becomes particularly severe at night. Preferred regions of infestation are the areas where the skin is thin (Deutsches Ärzteblatt International 2016; 113:757-62/Supplementary material).

As the mites are localised in the stratum corneum, in the majority of the cases it is sufficient a topical treatment with an antiscabies to kill the mites, the larves and the eggs. One of the drugs of choice for the treatment of scabies is permethrin, as it is effective against different kinds of scabies independently from the age of the patients. On the market a cream 5% permethrin is available. The 5% permethrin cream contains 0.45g benzyl alcohol per tube, which may cause allergic reactions, such as allergic contact dermatitis. Permethrin is also available in the market as a solution InfectoPedicul® 430mg/100ml for a one time treatment of the hair in case of head louses. The solution comprises among others ethanol 96%, 2-propanol and water.

Crotamiton is a scabicidal and antipruritic agent available as a cream or lotion for topical use only. It is a colorless to slightly yellowish oil, having a faint amine-like odor. It is miscible with alcohol and with methanol. Crotamiton is toxic to the scabies mite. Crotamiton is available as 10% lotion, cream and ointment as well as a 5% gel. The treatment requires 3 to 5 days to be effective.

Benzylbenzoate represents another option for treatment of scabies. This substance presents a good acaricide and ovocide effect. Commercial products containing benzylbenzoate comprise with propyleneglycol and cetylstearylalcohol potential contactallergenes. Available is an emulsion of benzylbenzoate in two concentrations 25% and 10% (Antiscabiosum®). The emulsion is applied for three consecutive days on the skin and only on the fourth day it can be removed by showering.

Demodex is a genus of tiny mites that live in or near hair follicles of mammals. Two species live on humans: *Demodexfolliculorum* and *Demodex brevis,* both frequently referred to as eyelash mites. Both species are primarily found in the face, near the nose, the eyelashes, and eyebrows, but also occur elsewhere on the body. The mites may be eradicated with topical insecticides such as crotamiton cream, permethrin cream, and also with topical or systemic metronidazole.

Lice are barely visible wingless insects that infest the head, body, or pubic area and live by sucking blood. They spread easily from person to person by close contact and shared clothing and other personal items. Head lice and pubic lice live directly on the person, whereas body lice live on clothing and bedding.

To treat head lice non-prescription shampoos and creams containing pyrethrins plus piperonyl butoxide are applied for 10 minutes and are then rinsed out. Prescription permethrin (a synthetic form of pyrethrin), applied as a liquid or as a cream, is also effective. Lindane as a prescription drug can be applied as a lotion or shampoo. It also cures lice infestation but is not as effective as the other preparations.

All of these louse treatments are repeated in 7 to 10 days to kill newly hatched lice. Lice have started to become resistant to drugs and may be hard to kill. One dose of the drug ivermectin is usually given by mouth if lice resist standard treatment. Lice that affect the eyelashes can be treated with petroleum jelly applied for 8 to 10 days, fluorescein eye drops, ivermectin taken by mouth, petrolatum salve, physostigmine ointment, or careful removal of each louse with an instrument.

One of the disadvantages of the commercial antiparasitic compositions is that they comprise alcohol which causes additional irritation and pain to the skin, in particular in presence of lesions.

EP2286663B1 describes alcohol free compositions comprising isopropyl myristate for use in methods of killing ectoparasites on a subject.

### Summary of the invention

The objective of the present invention is to provide an antiparasitic composition for topical application to the skin of a subject and which may be used to treat parasitic infestations such as scabies, louses and ticks.

The object of the present invention is attained according to the claims. The present invention provides an antiparasitic composition comprising an antiparasitic agent and a semifluorinated alkane.

In a second aspect, the present invention provides an antiparasitic composition comprising an antiparasitic agent and a semifluorinated alkane for use as a medicament, particularly for use in a method of treating a parasitic infestation.

In a third aspect, provided is also a method of topical application of an antiparasitic composition comprising applying with a wipe, a roll-on dispenser or a spray dispenser, an antiparasitic composition comprising an antiparasitic agent and a semifluorinated alkane.

In a fourth aspect, provided is a method of treating a parasitic infestation comprising topically applying an antiparasitic composition comprising an antiparasitic agent and a semifluorinated alkane to the skin of a subject in need thereof.

In a fifth aspect, the present invention provides a kit comprising a) an antiparasitic composition comprising an antiparasitic agent and a semifluorinated alkane and b) a dispenser selected from a roll-on dispenser, a spray dispenser and a wipe.

The present inventors have found that an antiparasitic composition can be obtained by mixing an antiparasitic agent and a semifluorinated alkane. Said composition has the advantages of being free of alcohol, of being easy to spread on the skin, for example through a roll-on dispenser, a wet wipe or a spray dispenser, therefore simplifying the application process to the skin of a subject. The composition of the present invention has the further advantage of not leaving greasy residues on the skin.

The composition of the present invention has the further advantage of allowing coverage of great or small areas of the skin easily and in a short time when applied through a roll-on dispenser or a spray dispenser or a wet wipe.

### Detailed description of the invention

The present invention provides an antiparasitic composition comprising an antiparasitic agent and a semifluorinated alkane.

An antiparasitic agent is a drug used to treat parasitic diseases, such as those caused for example by ectoparasites and parasitic fungi. In a preferred embodiment of the present invention, the antiparasitic agent is an ectoparasiticidal agent. An ectoparasiticide is an antiparasitic drug used in the treatment of ectoparasitic infestations, that is drugs used to kill the parasites that live on the body surface. Preferably, the antiparasitic agent is an ectoparasiticide selected from the group consisting of a pyrethroid, pyrethrins, an anilide, a benzoate, more preferably a pyrethroid, a benzoate or an anilide, most preferably a pyrethroid or an anilide.

Anilides (or phenylamides) are a class of chemical compounds which are acyl derivatives of aniline. Pyrethroids are synthetic chemical insecticides whose chemical structures are adapted from the chemical structures of the pyrethrins, which are botanical insecticides derived from chrysanthemum flowers.

In a preferred embodiment, the antiparasitic agent is selected from permethrin, benzyl benzoate, and crotamiton. More preferably, the antiparasitic agent is selected from permethrin and crotamiton, most preferably permethrin.

The antiparasitic agent may be present at a concentration of up to 10% w/v, preferably of up to 5 % w/v with respect to the total volume of the composition. In a preferred embodiment, the antiparasitic agent is present at a concentration of from 0.5% w/v to 10% w/v, preferably of from 0.5% w/v to 5% w/v with respect to the total volume of the composition.

Unless otherwise indicated, the term "% w/v" as used throughout herein in connection with the present composition denotes the amount of a component of a composition (such as, for example, the antiparasitic agent) as a weight percentage in relation to the total volume of the composition (with 'w' denoting the weight and 'v' denoting volume). For example, 0.05 % (w/v) may be understood as relating to 0.5 mg of a component in 1 mL of the composition, and 0.1 % (w/v) would correspond to 1.0 mg of a component in 1 mL of the composition.

The term "semifluorinated alkane", also referred to as "SFA" throughout this document, as used herein refers to a linear or branched compound composed of at least one perfluorinated segment (F-segment) and at least one non-fluorinated hydrocarbon segment (H-segment). Preferably, the semifluorinated alkane is a linear or branched compound composed of one perfluorinated segment (F-segment) and one non-fluorinated hydrocarbon segment (H-segment). Preferably, said semifluorinated alkane is a compound that exists in a liquid state within the temperature range of 4° to 40°C.

It is preferred that the F- and the H-segment of the linear or branched semifluorinated alkane comprise, independently from one another, 2 to 10 carbon atoms. According to a preferred embodiment of the present invention, the semifluorinated alkane is a linear compound of the formula (F(CF2)n(CH2)mH, wherein n and m are integers independently selected from each other from the range of 2 to 10.

According to another nomenclature, the linear semifluorinated alkane may be referred to as FnHm, wherein F means the perfluorinated hydrocarbon segment, H means the non-fluorinated hydrocarbon segment and n, m is the number of carbon atoms of the respective segment. For example, F4H5 is used for 1-perfluorobutyl-pentane. In a preferred embodiment of the present invention, the semifluorinated alkane is a semifluorinated alkane of formula F(CF2)n(CH2)mH wherein n is selected from 4 to 6 and m is selected from 5 to 10. More preferred is a semifluorinated alkane selected from the group consisting of F4H5, F4H6, F4H8, F4H10, F6H6, F6H8, F8H8, F6H10. Most preferred is a semifluorinated alkane selected from F4H8, F4H5, F6H6 and F6H8.

In the present invention the composition may comprise a semifluorinated alkane in an amount of from about 75 % (w/w) to about 99 % (w/w), more preferably from about 90 % (w/w) to about 99 % (w/w) with respect to the total weight of the composition. In a most preferred embodiment of the present invention, the composition comprises a semifluorinated alkane in an amount of at least 75 % w/w, preferably at least 85 % w/w, more preferably at least 90 % w/w with respect to the total weight of the composition.

In a preferred embodiment, the composition of the present invention is a liquid composition, that is the antiparasitic agent is dissolved or suspended in the semifluorinated alkane. In a most preferred embodiment, the composition of the present invention is a solution.

The antiparasitic composition of the present invention is preferably formulated as a solution, thus allowing application of the composition to the skin with for example a wet wipe, a roll-on dispenser or a spray dispenser. A wet wipe may comprise a substrate like a nonwoven material impregnated with the antiparasitic composition. The wet wipe is particularly useful in cases in which the area to be treated is small, such as the face, the scalp, the area around the eyes, the eyebrows, the eyelashes.

Alternatively, the antiparasitic composition of the present invention may be placed in a dispenser like a roll-on dispenser or a spray dispenser. The application of the antiparasitic composition of the present invention through a roll-on dispenser or a spray dispenser, may result useful in cases where the entire body or large areas of the body have to be treated as well as in cases where localized administration to small areas of the skin for example is required.

Preferably, the composition of the present invention is in the form of a wet wipe, a roll-on or a spray composition. Herein, a composition in form of a wet wipe, a roll-on or a spray, relates to a composition that is formulated in such way that it is suitable to be used or contained in devices, such as a wet wipe, a roll-on dispenser or a spray dispenser. More preferably, the composition of the present invention is in the form of a roll-on or spray composition. Even more preferably, the composition of the present invention is in form of a spray composition.

The antiparasitic composition of the present invention is preferably free of alcohol. The absence of alcohol constitutes a further advantage of the composition of the present invention, since alcohol causes further irritation or allergic reactions to the skin. In a preferred embodiment, the composition of the present invention is free of alcohol and/or preservative and/or water. In a preferred embodiment, the antiparasitic composition of the present invention is free of water, alcohol and preservatives.

The composition of the present invention may also comprise one or more further excipients as an additional component. The term "excipients" as used herein refers to any pharmaceutically acceptable natural or synthetic substance that may be added to the composition of the present invention. The present composition may comprise one or more excipients such as, for example, an antioxidant, a preservative, a lipid or oily excipient, a cosolvent, a surfactant or a lubricant or a combination thereof.

Suitable antioxidants which may be employed comprise, for example: butylated hydroxytoluene (BHT), butylated hydroxyanisole (BHA), tertiary butylhydroquinone (TBHQ), vitamin E, vitamin E derivatives (i.e. alpha-tocopherol acetate) and/or ascorbic acid or derivatives thereof.

Suitable oily excipients which may be added in the composition of the present invention comprise, for example, vegetable oils (i.e. soybean oil, olive oil, sesame oil, cotton seed oil, castor oil, sweet almond oil), mineral oil (i.e. petrolatum and liquid paraffin), medium chain triglycerides (MCT), oily fatty acids, squalane, squalene, essential oils, silicone oils, isopropyl myristate, oily fatty alcohols, esters of sorbitol and fatty acids, oily sucrose esters, or any other oily substance which is physiologically tolerated by the skin. In a preferred embodiment, the composition of the present invention comprises an oily excipient selected from squalane, silicone oils, mineral oils, essential oils, liquid triglycerides, medium chain tryglicerides, vegetable oils; preferably squalane, paraffin oil, medium chain tryglicerides. In a preferred embodiment, the composition of the present invention further comprises an oily excipient, preferably an oily excipient selected from squalane, mineral oil and medium chain tryglicerides. The oily excipient may be present in an amount of from 0 to 25 % w/w, preferably of from 0 to 10 % w/w, more preferably of from 0 to 5% w/w with respect to the total weight of the composition. Preferably the composition according to the invention further comprises an oily excipient selected from squalane, paraffin oil and medium chain tryglicerides at a concentration of from 0 to 25 % w/w, more preferably of from 0 to 10 % w/w, most preferably of from 0 to 5 % w/w with respect to the total weight of the composition.

In a second aspect, the present invention provides an antiparasitic composition comprising an antiparasitic agent and a semifluorinated alkane for use as a medicament, particularly for use in a method of treating a parasitic infestation. Preferably, the parasitic infestation is an ectoparasitic infestation, more preferably a mite infestation or a lice infestation, most preferably an infestation selected from scabies, head lice infestation, body lice infestation, mite infestation of the eyelashes and of the eyebrows (such as infestations by demodex mites), lice infestation of the eyelashes and of the eyebrows.

All the embodiments described above in connection with the first aspect of the invention apply to the antiparasitic composition for use as a medicament and for use in a method of treating a parasitic infestation, respectively.

In a third aspect, provided is also a method of topical application of an antiparasitic composition comprising applying with a wipe, a roll-on dispenser or a spray dispenser, an antiparasitic composition comprising an antiparasitic agent and a semifluorinated alkane. Preferably the method is applied to the skin of a subject in need thereof.

Unless otherwise indicated, a roll-on or roll-on dispenser as used in the present invention means a dispensing device comprising a container with a moving applicator ball mounted thereon, used for storing and application of a composition, contained in the container, to the body of a subject or onto any other surfaces. A spray dispenser as used herein means a dispensing device that turns a liquid into a fine mist, such as a spray bottle with a pump. A wipe or a wet wipe as used herein means a moist cloth.

All the embodiments described above in connection with the first aspect of the invention apply to the method of topical application of an antiparasitic composition of the third aspect of the invention
In a fourth aspect, provided is a method of treating a parasitic infestation comprising topically applying an antiparasitic composition comprising an antiparasitic agent and a semifluorinated alkane to the skin of a subject in need thereof. In a preferred embodiment, the method of treating a parasitic infestation comprises the step of applying the antiparasitic composition of the present invention to the skin of a subject in need thereof with a roll-on dispenser, a spray dispenser or a wipe. In a more preferred embodiment, the method of treating a parasitic infestation comprises the step of applying the antiparasitic composition of the present invention to the skin of a subject in need thereof through a roll-on dispenser, a spray dispenser with a pump or a wipe.

Preferably, the parasitic infestation is an ectoparasitic infestation, more preferably a mite infestation or a lice infestation, most preferably an infestation selected from scabies, head lice infestation, body lice infestation, mite infestation of the eyelashes and of the eyebrows (such as infestations by demodex mites), lice infestation of the eyelashes and of the eyebrows.

All the embodiments described above in connection with the first aspect of the invention apply to the method of treating a parasitic infestation of the fourth aspect of the invention.

In a fifth aspect, the present invention provides a kit comprising a) an antiparasitic composition comprising an antiparasitic agent and a semifluorinated alkane and b) a dispenser selected from a roll-on dispenser, a spray dispenser and a wipe.

All the embodiments described above in connection with the first aspect of the invention apply to the kit of the fifth aspect of the invention.

The followings are numbered items comprised by the present invention:
1. An antiparasitic composition comprising an antiparasitic agent and a semifluorinated alkane.
2. The antiparasitic composition of item 1 in form of a liquid composition, preferably a solution.
3. The antiparasitic composition of any of the preceding items, wherein the antiparasitic agent is an ectoparasiticide.
4. The antiparasitic composition of any of the preceding items wherein the antiparasitic agent is one selected from the group consisting of a pyrethroid, a pyrethrin, an anilide, a benzoate.
5. The antiparasitic composition of any of the preceding items wherein the antiparasitic agent is selected from the group consisting of a pyrethroid, a benzoate or an anilide,
6. The antiparasitic composition of any of the preceding items wherein the antiparasitic agent is selected from the group consisting of a pyrethroid or an anilide.
7. The antiparasitic composition of any of the preceding items wherein the antiparasitic agent is selected from permethrin, crotamiton and benzylbenzoate.
8. The antiparasitic composition of any of the preceding items, wherein the semifluorinated alkane is of generic formula F(CF2)n(CH2)mH, wherein n and m are integers independently selected from each other from the range of 2 to 10.
9. The antiparasitic composition of any of the preceding items, wherein the semifluorinated alkane is of formula F(CF2)n(CH2)mH wherein n is selected from 4 to 6 and m is selected from 5 to 10.
10. The antiparasitic composition of any of the preceding items, wherein the semifluorinated alkane is selected from the group consisting of F4H5, F4H6, F4H8, F4H10, F6H6, F6H8, F8H8, F6H10.
11. The antiparasitic composition of any of the preceding items, wherein the semifluorinated alkane is selected from F4H8, F4H5, F6H6 and F6H8.
12. The antiparasitic composition of any of the preceding items, wherein the semifluorinated alkane is selected from F4H8, F6H6 and F6H8.
13. The antiparasitic composition of any of the preceding items, wherein the semifluorinated alkane is F6H8.
14. The antiparasitic composition of any of the preceding items, wherein the antiparasitic agent is present at a concentration of from 0.5% w/v to 10% w/v with respect to the total volume of the composition.
15. The antiparasitic composition of any of the preceding items, wherein the antiparasitic agent is present at a concentration of from 0.5% w/v to 5% w/v with respect to the total volume of the composition.
16. The antiparasitic composition of any of the preceding items, wherein the composition further comprises an oily excipient.
17. The antiparasitic composition of any of the preceding items, wherein the composition further comprises an oily excipient selected from squalane, silicone oils, mineral oils, essential oils, liquid triglycerides, medium chain tryglicerides, vegetable oils.
18. The antiparasitic composition of any of the preceding items, further comprising an oily excipient selected from squalane, paraffin oil, medium chain tryglicerides.
19. The antiparasitic composition of any of the preceding item further comprising squalane.
20. The antiparasitic composition of any of items 16 to 19, wherein the oily excipient is present at a concentration of from 0 to 25% w/w with respect to the total weight of the composition.
21. The antiparasitic composition of item 20, wherein the oily excipient is present at a concentration of from 0 to 10 % w/w with respect to the total weight of the composition.
22. The antiparasitic composition of any of items 1 to 15 wherein the composition consists of an antiparasitic agent and a semifluorinated alkane.
23. The antiparasitic composition of any of items 1 to 21, wherein the composition consists of an antiparasitic agent, a semifluorinated alkane and an oily excipient.
24. The antiparasitic composition of any of the preceding items, wherein the composition is free of alcohol.
25. The antiparasitic composition of any of the preceding items, wherein the composition is free of water.
26. The antiparasitic composition of any of the preceding items, wherein the composition is free of preservatives.
27. The antiparasitic composition according to any of the preceding items in the form of a wet wipe, a roll-on or a spray composition.
28. The antiparasitic composition as defined in any of the preceding items, for use as a medicament.
29. The antiparasitic composition as defined in any of the preceding items for use in a method of treating a parasitic infestation.
30. The antiparasitic composition for the use of item 29, wherein the parasitic infestation is selected from a mite infestation and a lice infestation.
31. The antiparasitic composition for the use of item 30, wherein the parasitic infestation is selected from scabies, head lice infestation, body lice infestation, lice infestation of the eyelashes and of the eyebrows, mite infestations of the eyelashes and of the eyebrows.
32. The antiparasitic composition for the use of item 31, wherein the parasitic infestation is scabies.
33. The antiparasitic composition for the use of any of items 29 to 32, wherein the antiparasitic agent is permethrin.
34. A method of topical application of an antiparasitic composition comprising applying with a wipe, a roll-on dispenser or a spray dispenser an antiparasitic composition as defined in any of items 1 to 27.
35. A method of treating a parasitic infestation comprising topically applying an antiparasitic composition as defined in any of items 1 to 27 to the skin of a subject in need thereof or to a surface contaminated by parasites.
36. The method according to item 35, wherein the antiparasitic composition is applied with a roll-on dispenser, a spray dispenser or a wipe.
37. A kit comprising a) an antiparasitic composition according to any of items 1-27, b) a wipe, a spray dispenser or a roll-on dispenser.
38. A kit according to item 37, further comprising instructions for use.

The following examples serve to illustrate the invention; however, these are not to be understood as restricting the scope of the invention.

### Examples

### Example 1

### Preparation of a solution of permethrin 5% w/v in F4H8.

250 mg permethrin (Dr. Reddy's, Ph.Eur. grade, cis:trans 25:75) is weighed in a 5-ml volumetric flask; the flask is then filled to 5 ml with F4H8 (Novaliq, purity >99%), closed and shaken at 280 rpm, RT for 16 h. The resulting 50 mg/ml solution appears clear.

### Example 2

### Preparation of a solution of crotamiton 10% w/v in F6H8

500 mg crotamiton (SynTech, Ph.Eur. grade) is weighed in a 5-ml volumetric flask; the flask is then filled to 5 ml with F6H8 (Novaliq, purity >99%), closed and shaken for 5 min at 280 rpm, RT. The resulting 100 mg/ml solution appears clear.

### Example 3

### Preparation of a solution of benzylbenzoate in F4H5

500 mg benzyl benzoate (Symrise, puritiy >99%) is weighed in a 5-ml volumetric flask; the flask is then filled to 5 ml with F4H5 (Novaliq, purity >99%), closed and shaken for 5 min at 280 rpm, RT. The resulting 100 mg/ml solution appears clear.

### Example 4

Following the procedure described in the Examples 1 to 3, the following solubility tests were carried out, as shown in Table 1, Table 2 and Table 3.

**Table 1: Permethrin solubility tests**

| Solvent | Solubility (% w/v) |
|---|---|
| F6H8 | 3.9% |
| F4H5 | 4.9% |
| F4H8 | 10.1% |
| 10% MCT in F6H8 | 4.8% |
| 10% MCT in F4H5 | 6.9% |
| 10% paraffin oil in F6H8 | 4.0% |
| 10% paraffin oil in F4H5 | 7.4% |
| F6H8-ene | 5.5% |
| F6H2 | 0.42% |

**Table 2: Crotamiton solubility tests**

| Solvent | Solubility (%w/v) |
|---|---|
| F6H8 | >12% |
| F4H5 | >12% |
| F6H2 | ∼6% |

**Table 3: Benzylbenzoate solubility tests**

| Solvent | Solubility (%w/v) |
|---|---|
| F6H8 | 9-10 % |
| F4H5 | 11-11.5% |

## Claims

1. An antiparasitic composition comprising an antiparasitic agent and a semifluorinated alkane.

2. The antiparasitic composition of claim 1, that is a liquid composition, preferably a solution.

3. The antiparasitic composition of any of the preceding claims, wherein the antiparasitic agent is one selected from a pyrethroid, a pyrethrin, an anilide, a benzoate.

4. The antiparasitic composition of any of the preceding claims, wherein the antiparasitic agent is one selected from permethrin, crotamiton and benzylbenzoate.

5. The antiparasitic composition of any of the preceding claims, wherein the semifluorinated alkane is of generic formula F(CF2)n(CH2)mH, wherein n and m are integers independently selected from each other from the range of 2 to 10.

6. The antiparasitic composition according to any of the preceding claims in the form of a wipe, a roll-on or a spray composition.

7. The antiparasitic composition according to any of the preceding claims, wherein the composition is free of alcohol.

8. The antiparasitic composition according to any of the preceding claims for use as a medicament.

9. The antiparasitic composition as defined in any of the preceding claims for use in a method of treating a parasitic infestation.

10. The antiparasitic composition for the use of claim 9, wherein the parasitic infestation is selected from a mite infestation and a lice infestation.

11. The antiparasitic composition for the use of claim 10, wherein the parasitic infestation is selected from scabies, head lice, body lice, lice of the eyelashes, lice of the eyebrows, mites of the eyelashes or mites of the eyebrows.

12. The antiparasitic infestation of any of the preceding claims, wherein the antiparasitic agent is permethrin.

13. A method of topical application of an antiparasitic composition comprising applying with a wipe, a roll-on dispenser or a spray dispenser an antiparasitic composition as defined in any of claims 1 to 12.

14. A kit comprising a) an antiparasitic composition according to any of claims 1-12, b) a wipe, a spray dispenser or a roll-on dispenser.
